Europäisches Patentamt

European Patent Office

Office européen des brevets

(11) Veröffentlichungsnummer: **0 376 891**

**A1**

(12) **EUROPÄISCHE PATENTANMELDUNG**

(21) Anmeldenummer: 89810981.4

(22) Anmeldetag: 22.12.89

(51) Int. Cl.⁵: **A61K 9/20, A61K 9/32**

Patentansprüche für folgende Vertragsstaaten:
ES + GR

(30) Priorität: 30.12.88 CH 4855/88

(43) Veröffentlichungstag der Anmeldung:
04.07.90 Patentblatt 90/27

(84) Benannte Vertragsstaaten:
**AT BE CH DE ES FR GB GR IT LI LU NL SE**

(71) Anmelder: **CIBA-GEIGY AG**
**Klybeckstrasse 141**
**CH-4002 Basel(CH)**

(72) Erfinder: **Khanna, Satish Chandra, Dr.**
**Rütistrasse 26**
**CH-4103 Bottmingen(CH)**

(54) **Überzogene Klebetabletten.**

(57) Gegenstand der Erfindung ist eine pharmazeutische Zusammensetzung für die Applikation von Baclofen auf den Schleimhäuten in der Mundhöhle in Form einer Klebetablette. Diese ist gekennzeichnet durch:

    a) einen hydrophilen Tablettenkern, welcher mit seiner Oberfläche an der Applikationsfläche der Mundschleimhaut haftet und den Wirkstoff Baclofen, ein quellfähiges Vinylpolymerisat, ein Galactomannan und/oder ein pharmazeutisch verwendbares Wachs und/oder ein gegebenenfalls vollständig oder partiell hydriertes Glycerid und gegebenenfalls weitere pharmazeutisch verwendbare Hilfsstoffe enthält, und gegebenenfalls

    b) einen hydrophoben Ueberzug, welcher den Tablettenkern a) mit Ausnahme der für die Abgabe von Baclofen vorgesehenen Fläche umhüllt.

Die Erfindung betrifft ausserdem ein Verfahren zur Herstellung dieser Klebetablette und ihre therapeutische Verwendung als Spasmolytikum.

EP 0 376 891 A1

## Ueberzogene Klebetabletten

Gegenstand der Erfindung ist eine pharmazeutische Zusammensetzung für die Applikation von Baclofen auf den Schleimhäuten in der Mundhöhle in Form einer Klebetablette, Verfahren zur Herstellung dieser Klebetablette und ihre therapeutische Verwendung als Spasmolytikum.

Baclofen: 4-Amino-3-(4-chlorphenyl)-buttersäure (Lioresal®: Ciba-Geigy) ist als Myotonolytikum u.a. bei Spastizität der Skelettmuskulatur bei multipler Sklerose und auch infektiös, degenerativ, traumatisch oder neoplastisch bedingter Rückenmarkserkrankung, Querschnittsmyelitis etc therapeutisch verwendbar, siehe Rote Liste Nr. 63008, Verzeichnis von Fertigarzneimitteln, Editio Cantor 1987, D-7960 Aulendorf. Handelsübliche Darreichungsformen sind Tabletten mit 5, 10 und 25 mg Wirkstoffgehalt.

Bei Beginn der Therapie mit den üblichen Tabletten kann es zu Tagessedation, Uebelkeit, Erbrechen, Durchfällen und Schwindel, gelegentlich, insbesondere bei älteren Patienten, zu psychotischen Erscheinungen wie depressiver Verstimmung kommen, siehe Rote Liste loc. cit.. Wegen solcher Nebenwirkungen wird eine sogenannte einschleichende Dosierung vorgenommen, z.B. bei Erwachsenen zu Beginn 3 x täglich 5 mg, Steigerung der Einzeldosis alle 3 Tage um jeweils 5 mg bis zum Richtwert von 30-75 mg täglich.

Solche Dosierungsvorschriften sind aus mehreren Gründen nachteilig. Durch den mehrtägigen Anstieg der Anzahl der Verabreichungen zu Beginn der Therapie sowie die häufigen Verabreichungen in deren Verlauf besteht das Risiko von Dosierungsfehlern durch irrtümliche Anwendung der Vor schriften, z.B. durch Unterlassung oder verfrühte Einnahme einer zu hohen Dosis. Es kann daher der Fehler der Ueber- als auch der Unterdosierung auftreten.

Der vorliegenden Erfindung liegt die Aufgabe zugrunde, speziell für Baclofen eine verbesserte Darreichungsform herzustellen, die eine gleichmässige Applikation sowohl zu Beginn als auch im weiteren Verlauf der Therapie in gleichbleibenden zeitlichen Abständen ermöglicht. Mit der verbesserten Darreichungsform soll auch die Anzahl der täglichen Verabreichungen, vorzugsweise auf ein bis zwei, und die Dosis selbst reduziert werden.

Zur Lösung dieser Aufgabenstellung eignen sich auch keine anderen Formulierungen wie Kapseln, Dragees oder Sirupe, da nach Verabreichung mit diesen enteralen Darreichungsformen genügende Resorption von Baclofen nur im oberen Gastrointestinaltrakt erfolgt, insbesondere im Duodenum. Erfolgt neben rascher Resorption auch noch eine schnelle Metabolisierung des Wirkstoffs, lässt sich die Anzahl der Verabreichungen und auch die Dosierung nicht reduzieren.

Die Verabreichung über die Mundschleimhäute mittels sogenannter bukkaler oder sublingualer Formulierungen wie Klebestreifen (lozenges) oder Klebetabletten bietet den Vorteil einer teilweisen oder weitgehenden Umgehung der unteren Teile des Gastrointestinaltrakts sowie einer verlängerten Wirkstoffabgabe in der Mundhöhle.

Eine geeignete Formulierung für Baclofen hätte eine kontinuierliche Abgabe des Wirkstoffs über einen längeren Zeitraum von mehr als 12 Stunden bei der üblichen Dosierung von 25 mg bzw. mehr als 6 Stunden bei geringeren Dosierungen zu gewährleisten, so dass sich durch diese Darreichungsform die Zahl der Applikationen bei gleicher Dosis bzw. die Dosis bei gleichbleibender Applikation reduzieren liesse.

Formulierungen in Form von Klebestreifen können nachteilig sein, da diese auf den Schleimhäuten in der Mundhöhle eine grosse Applikationsfläche einnehmen und als Fremdkörper empfunden werden. Klebetabletten haben den Vorteil einer kleineren Applikationsfläche. Allerdings müssen solche Darreichungsformen auf dieser kleineren Fläche ausreichende Adhäsionseigenschaften besitzen, so dass sie selbst beim Kauen oder Essen sowie bei mechanischer Bewegung der Zunge an der Mundschleimhaut haften bleiben und sich trotzdem, falls nötig, mühelos entfernen lassen.

Diese Aufgabe wird durch die vorliegende Erfindung gelöst, welche eine pharmazeutische Zusammensetzung für die Applikation von Baclofen auf den Schleimhäuten in der Mundhöhle in Form einer Klebetablette betrifft. Diese pharmazeutische Zusammensetzung ist gekennzeichnet durch

a) einen hydrophilen Tablettenkern, welcher mit seiner Oberfläche an der Applikationsfläche der Mundschleimhaut haftet und den Wirkstoff Baclofen oder ein pharmazeutisch verwendbares Salz davon und als Hilfsstoffe ein quellfähiges Vinylpolymerisat, ein Galactomannan und/oder ein pharmazeutisch verwendbares Wachs und/oder ein gegebenenfalls vollständig oder partiell hydriertes Glycerid und gegebenenfalls weitere pharmazeutisch verwendbare Hilfsstoffe enthält, und gegebenenfalls

b) einen hydrophoben Ueberzug, welcher den Tablettenkern a) mit Ausnahme der für die Abgabe von Baclofen vorgesehenen Fläche umhüllt.

Diese pharmazeutische Zusammensetzung zeichnet sich durch hervorragende Klebe- und Abgabeeigenschaften aus. Wie humane Bioverfügbarkeitsdaten zeigen, steigen im Verlauf der Zeit nach Einnahme der Darreichungsform die Plasmakonzentrationen weniger stark als nach Einnahme von konventionellen

2

Tabletten an. Die Wirkstoffkonzentration bleibt aber länger auf therapeutischem Niveau erhalten. Der Abfall der Wirkstoffkonzentrationen erfolgt zu einem späteren Zeitpunkt. Wegen ihrer geringen Grosse lässt sich die pharmazeutische Zusammensetzung an beliebigen Stellen der Mundhöhle auf den Schleimhäuten applizieren, insbesondere am Gaumen, aber auch bukkal, lingual oder sublingual, und behindert durch ihren festen Sitz die Nahrungsaufnahme nicht. Durch gute Geschmackseigenschaften, geringe Grösse und Elastizität in gequollenem Zustand wird die Klebetablette nicht als "Fremdkörper" empfunden. Ihre Anwesenheit im Mundraum entfällt dem Bewusstsein und wird sogar "vergessen". Mit der Klebetablette lässt sich die Anzahl der Verabreichungen reduzieren. Bei einer Dosis von 25 mg genügen ein bis zwei Verabreichungen pro Tag, denn es erfolgt eine kontinuierliche Wirkstoffzufuhr. Auch nachts ist die Abgabe von therapeutisch wirksamen Mengen gewährleistet.

Die weiter vorn und im folgenden verwendeten Begriffe und Ausdrücke sind im Rahmen der Beschreibung der Erfindung wie folgt definiert: Der hydrophile Tablettenkern ist klebefähig und zur Aufnahme von Wasser bzw. Speichel befähigt.

Ein Teil des Wirkstoffs wird an diese Flüssigkeit abgegeben und gelangt mit dieser in den Gastrointestinaltrakt. Ein weiterer Anteil gelangt durch die Schleimhäute direkt in den Blutkreislauf.

Ein pharmazeutisch verwendbares Salz von Baclofen ist insbesondere ein Additionssalz mit einer geeigneten Säure, z.B. verdünnter, wässriger Halogenwasserstoffsäure, z.B. Chlor- oder Bromwasserstoffsäure. Als Additionssalz ist das Hydrochlorid bevorzugt. Die Herstellung von Baclofen und seiner Salze ist in der U.S. Patentschrift 3,634,428 beschrieben.

Ein quellfähiges Vinylpolymerisat dehnt sich durch die Einwirkung von Wasser bzw. Speichel bei der Körpertemperatur aus, ist elastisch und beim pH-wert der Speichelflüssigkeit wasserlöslich. Der Lösungsvorgang setzt langsam bei und nach der rasch erfolgenden Quellung ein. In Abhängigkeit von der Zusammensetzung und dem Polymerisationsgrad des verwendeten Vinylpolymerisats löst sich dieses teilweise oder ganz während des Haftens an der Mundschleimhaut auf.

Ein quellfähiges Vinylpolymerisat ist insbesondere ein hydrophiles Polymeres der Acrylsäure, der Methacrylsäure und Ester davon, ein Copolymeres der Acrylsäure, Methacrylsäure und der Ester davon, ein Copolymeres der Methacrylsäure und der Ester davon, ein Copolymeres von Methacrylsäureestern oder ein polymeres, aliphatisches oder zyklisches Vinylamid.

Ein hydrophiles Polymeres der Acrylsäure hat vorzugsweise eine mittlere Molmasse von ca. $8,0 \times 10^5$ bis $1,0 \times 10^6$ und wird in der Pharmazie als Hilfsstoff unter dem Freinamen Carbomer verwendet. Besonders bevorzugt sind Polymere, welche von der Fa. Goodrich unter der Bezeichnung CARBOPOL 934 vertrieben werden und pharmazeutische Qualität besitzen, z.B. CARBOPOL 934 P.

Hydrophile Polymere der Methacrylsäure und Ester davon, Copolymere der Methacrylsäure und Ester davon sowie Copolymere von Methacrylsäureestern haben vorzugsweise eine mittlere Molmasse von ca. $1,0 \times 10^5$ bis $1,0 \times 10^6$. Die Säuregruppen sind teilweise oder vollständig durch $C_1$-$C_4$-Alkylgruppen ersetzt, insbesondere Methyl- oder Aethylgruppen, wobei diese Estergruppen ihrerseits durch hydrophile Gruppen, insbesondere Trimethylammoniumäthyl, substituiert sein können.

Solche Polymere sind unter dem registrierten Warenzeichen EUDRAGIT der Fa. Röhm Pharma Weiterstadt, Bundesrepublik Deutschland, erhältlich. Besonders bevorzugt sind quellbare, permeable Typen wie EUDRAGIT NE 30 D. Bei Verwendung von Typen wie EUDRAGIT L, S, RN oder RS lässt sich eine Abgabeverzögerung erreichen.

Ein quellfähiges Vinylpolymerisat ist ferner ein polymeres aliphatisches oder zyklisches Vinylamid, z.B. Poly-N-vinylmethylacetamid, -äthylacetamid, -methylpropionamid, -äthylpropionamid, -methylisobutyramid, -2-pyrrolidon, -2-piperidon, -epsilon-caprolactam, -5-methyl-2-pyrrolidon oder -3-methyl-2-pyrrolidon, oder Poly-N-vinyl-2-pyrrolidon mit einer mittleren Molmasse von ca. 10'000 bis 700'000, oder ein Copolymeres aus den genannten aliphatischen und zyklischen Vinylamiden, z.B. das Copolymere von Vinyl-2-pyrrolidon und Vinylacetat oder ein Hydrolyseprodukt davon, z.B. das Vinyl-2-pyrrolidon-Vinylalkohol-Copolymerisat mit einem Hydrolysegrad von ca. 85-98 % des Vinylalkoholbestandteils.

Bevorzugt ist Poly-N-vinyl-2-pyrrolidon mit einer mittlere Molmasse von 20'000 bis 700'000, welches kommerziell unter den Bezeichnungen Kollidon® (BASF) erhältlich ist und z.B. folgende Eigenschaften hat:

Löslich in Wasser, Ethanol, Methanol, Isopropanol, Glycerin, Propylenglycol, Methylenchlorid, unlöslich in Ether, Kohlenwasserstoffen, stark hygroskopisch (Wasseraufnahme von ca. 33 % ab ca. 70 % rel. Luftfeuchtigkeit), siehe Pharmazeutische Technologie, Sucker H. et al., Thieme Verlag Stuttgart 1978, Seite 339.

Als quellfähiges Vinylpolymerisat ist ebenfalls das Vinylpyrrolidon-Vinylacetat-Copolymerisat mit einem Monomerenverhältnis Vinylpyrrolidon zu Vinylacetat von ca. 60:40 (Gew.- %) mit folgenden Eigenschaften bevorzugt:

Reinheit: 95 % (Rest Wasser), unlöslich in Ether, aliphatischen Kohlenwasserstoffen, sehr gut löslich in Wasser, Ethyl- und Isopropylalkohol, Methylenchlorid, Glycerin und 1,2-Propylenglycol, pH-Wert einer 10 %igen wässrigen Lösung: 3-5, Viskosität (10 %ige wässrige Lösung): 5 mPas, siehe H.P. Fiedler, Lexikon der Hilfsstoffe, Editio Cantor 1982.

Solche Vinylpyrrolidon-Vinylacetat-Copolymerisate sind bekannt und/oder auf an sich bekannte Weise in beliebigem Mischungsverhältnis der Monomeren herstellbar. Das 60:40-Copolymerisat ist z.B. kommerziell unter der Bezeichnung Kollidon® VA 64 (BASF) erhältlich.

Ein Galactomannan ist ein Polysaccharid, welches in den Endospermzellen von Leguminosen vorhanden ist und als pharmazeutischer Hilfsstoff verwendet wird. Bevorzugt ist ein kaltwasserlösliches Galactomannan (25° C) mit einer Brookfield-Viskosität höher als 1000 [mPa sec] (gemessen in demineralisierter wässriger Lösung bei 25° C, Hydratationszeit von 1-24 Stunden und Rühren mit 20 Upm = Umdrehungen pro Minute), welches unter Trivialnamen wie Johannisbrotkernmehl (Carubin, locust bean gum), Guar-Gummi (Guaran, guar gum) oder Tara-Gummi (tara gum) bekannt ist.

Besonders bevorzugt ist ein Galactomannan, welches unter dem Handelsnamen Meyprogat® von der Firma Meyhall, CH-8280 Kreuzlingen, vertrieben wird, insbesondere die Typen MEYPROGAT 30, 60, 90 und 120, und vor allem MEYPROGAT 150.

Ein pharmazeutisch annehmbares Wachs besteht im wesentlichen aus Gemischen bei Raumtemperatur fester oder halbfester Ester linearer Carbonsäuren mit ca. 18-34 C-Atomen mit etwa gleich langen linearen Alkoholen. Bevorzugt sind Wachse natürlichen, insbesondere pflanzlichen Ursprungs, welche die genannten Ester als Hauptbestandteile (mehr als 50 %) sowie als weitere Bestandteile die den Estern zugrunde liegenden freien Säuren und Alkohole, ferner Lactone, Lactide, Kohlenwasserstoffe, Sterine u.a. enthalten.

Ein pharmazeutisch annehmbares Wachs ist ferner ein bei Raumtemperatur flüssiger, synthetischer Ester linearer Carbonsäuren mit ca. 10-20 C-Atomen und etwa gleich langen linearen Alkoholen, ein Gemisch dieser synthetischen Ester, gegebenenfalls mit den genannten Wachsen natürlichen Ursprungs, Paraffinwachs mit mehr als 20 C-Atomen sowie ein unter der Bezeichnung Carbowax bekanntes synthetisches Polyäthylenglycolgemisch.

Wachse natürlichen Ursprungs sind beispielsweise unter der Bezeichnung Cera flava (Gelbes Wachs, Bienenwachs), Cera alba (gebleichtes Wachs), Cera subliquida (dickflüssiges Wachs), Propolis (Bienenharz), Carnaubawachs, chinesisches Wachs, Japanwachs, Afridiwachs, Candellilawachs, Gheddawachs, Godangwachs, Kapbeerenwachs, Myrtenwachs, Okubawachs, Pisangwachs, Raphiwachs oder Cetina bekannte Wachse tierischen oder pflanzlichen Ursprungs.

Ein bei Raumtemperatur flüssiger, synthetischer Ester linearer Carbonsäuren mit ca. 10-20 C-Atomen und etwa gleich langen linearen Alkoholen ist beispielsweise Oelsäureoleoylester (DAB 7) oder Oelsäuredecylester, z.B. die unter dem Warenzeichen CETIOL der Fa. Henkel vertriebenen Handelsformen, bzw. die unter den Bezeichnungen CETIOL A, B, LC und SN kommerziell erhältlichen Analoga.

Paraffinwachs (DAB 7) mit mehr als 20 C-Atomen besteht im wesentlichen aus einem Gemisch bei Raumtemperatur fester, gesättigter Kohlenwasserstoffe, vorzugsweise mit einer Kettenlänge bis zu 30 C-Atomen.

Carbowax ist die Bezeichnung für ein Gemisch synthetischer Polyäthylenglycole mit einem Molekulargewicht von mehr als 600, welche bei Raumtemperatur halbfest (salbenartig) oder wachsartig fest sind. Bevorzugt sind bei Raumtemperatur feste Carbowaxe mit einem Molekulargewicht oberhalb 4000.

Ein Glycerid besteht im wesentlichen aus einem Gemisch natürlichen Ursprungs oder aus einer isolierten natürlichen oder synthetischen, im wesentlichen reinen Einzelkomponente eines in diesem natürlichen Gemisch vorhandenen Esters des Glycerins mit einem (Monoglycerid), zwei (Diglycerid) oder drei (Triglycerid) Aequivalenten ungesättigter oder gesättigter Fettsäuren mit ca. 4 bis 30 C-Atomen, welche bei Raumtemperatur in flüssigem oder halbfestem, vorzugsweise festem, Zustand vorliegen.

Ein vollständig oder partiell hydriertes Glycerid besteht aus dem Gemisch natürlichen Ursprungs enthaltend Ester des Glycerins mit ungesättigten Fettsäuren oder aus einer synthetischen, im wesentlichen reinen Einzelkomponente eines in solchen natürlichen Gemischen vorhandenen Esters des Glycerins mit ungesättigten Fettsäuren, welche man den bekannten Hydrierungsbedingungen, z.B. katalytisch mit Wasserstoff, unterworfen hat.

Gemische natürlichen Ursprungs mit den genannten Glyceriden sind z.B. die trivial unter den Bezeichnungen Oele, Talge und Fette bekannten flüssigen, halbfesten und festen, gereinigten Gemische pflanzlichen, z.B. aus Saatgut oder Früchten, oder tierischen Ursprungs.

Solche Gemische sind z.B. die unter der Bezeichnung Olea piguia (neutralisierte fette Oele), insbesondere Oel für Injektionszwecke, Avocado-Oel, Babassuöl, Baumwollsamenöl, Borneo-Talg, Bucheckernöl, Erdnussöl, Hammeltalg, Haselnussöl, Hydnocarpus-Oele, Holzöl, Kakaobutter, Kapoköl, Kokosfett oder Kokosöl, Kürbiskernöl, Lebertran, Lorbeeröl, Maisöl, Mandelöl, Miglyolöle, Mohnöle, Mowrah-Butter, Olivenöl, Palmkernfett bzw. Palmkernöl, Palmöl, Pfirsichkernöl, Rapsöl, Ricinusöl, Rindertalg, Safloröl, Sesamöl,

4

Shea-Butter, Softisan, Sojaöl, Sonnenblumenöl, Teesamenöl oder Walnussöl in nationalen bzw. supranationalen Pharmakopöen, z.B. der europäischen Pharmakopöe, beschriebenen Produkte.

Bevorzugt ist ein vollständig oder partiell hydriertes Glycerid, z.B. erhältlich durch partielle oder vollständige Hydrierung der genannten natürlichen Gemische, z.B. die unter der Bezeichnung Gehärtetes Erdnussöl, Hartfett, z.B. Gemische von Mono-, Di- und Triestern von Palmitin-und Stearinsäure mit Glycerin, z.B. die unter dem Warenzeichen PRECIROL (Gattefossé SA, France) vertriebenen Produkte, oder gehärtetes Ricinusöl (hydrogenated Gastoroil), z.B. die unter den Warenzeichen CUTINA und CEROXIN (Henkel), CASTORWAX und OPALWAX (Baker Castor Oil Co. USA), CENWACHS (W.C. Hardesty, N.Y. USA) oder CETYNOL (Givaudan, Genève CH) kommerziell erhältlichen Produkte.

Weitere pharmazeutisch annehmbare Hilfsstoffe sind die bei der Tablettierung zur Herstellung von Granulaten verwendeten üblichen Hilfsstoffe, z.B. Bindemittel, Gleitmittel, Fliessmittel, Dispergiermittel, Füllmittel etc. So können übliche Hilfsstoffe wie Lactose, Saccharose, Sorbit, Mannit, oder Cellulose, insbesondere mikrokristalline Cellulose, oder Magnesiumstearat zusätzlich zu den genannten Hilfsstoffen verwendet werden.

Der hydrophobe Ueberzug b) umhüllt gegebenenfalls den Tablettenkern a) mit Ausnahme der für die Applikation vorgesehenen nicht bedeckten Fläche. Mit dieser überzugsfreien Fläche liegt die Klebetablette auf der Schleimhaut auf und ist dort befestigt. Die überzugsfreie Fläche befindet sich auf der Ober- oder Unterseite der Tablette und kann sich farblich von der Farbgebung des Ueberzuges unterscheiden.

Der hydrophobe Ueberzug selbst nimmt Körperflüssigkeit wie Speichelflüssigkeit im Gegensatz zum hydrophilen Kern nur langsam auf. Bei dessen Quellung dehnt sich der Ueberzug gleichförmig aus, so dass die neutralen Geschmackseigenschaften der Tablette, insbesondere das Gefühl der Weichheit und Glätte, erhalten und der Geschmack der Bestandteile des Tablettenkerns überdeckt bleibt.

Dadurch wird eine Anpassung der Klebetablette selbst in gequollenem Zustand an die Schleimhäute erreicht, so dass sie sich fast überall in der Mundhöhle applizieren lässt.

Der hydrophobe Ueberzug b) umhüllt den Tablettenkern a) vorzugsweise in Filmstärke, z.B. ca. 0,01 - 0,1 mm. Es eignen sich filmbildende, pharmazeutische annehmbare Wachse oder Polymere, die aus einer organischen Lösung heraus oder einer wässrigen Dispersion appliziert werden können und wasserunlösliche oder schlecht wasserlösliche, aber poröse Filme bilden können, z.B. die weiter vorn genannten Wachse in Filmschicht, Polyäthylenglycole, Aethylcellulose, Polyvinylacetat, Celluloseacetat, ein Gemisch von Polyvinylpyrrolidon bzw. Copolymerisat von Polyvinylpyrrolidon bzw. Copolymerisat von Polyvinylpyrrolidon und Polyvinylacetat mit Hydroxypropylmethylcellulose, polymere Epoxide, Copolymere aus Alkylenoxid und Alkylglycidyläthern, Polymilchsäurederivate u.ä.. Es eignen sich auch alle in der Literatur bekannten Filmschicht-Materialien (Membranen), die poröse Eigenschaften aufweisen, z.B. Mischungen von wasserunlöslichen Acrylaten, z.B. Copolymerisate von Acrylsäureestern und Methacrylsäureestern des Typs EUDRAGIT (Röhm Pharma), die in wässriger Dispersion aufgetragen werden.

Bevorzugt sind filmbildende wachsartige Polymere aus der Gruppe der Aethylenoxidhomopolymere mit der Bezeichnung Polyäthylenglycol (PEG) oder Polyäthylenoxid, insbesondere Polyäthylenglycol mit einer durchschnittlichen Molmasse von 2'000 bis 20'000, z.B. PEG 2'000, 3'000, 4'000, 6'000 und 8'000. Diese Polymere werden von verschiedenen Herstellern unter verschiedenen Handelsnamen vertrieben.

Der Ueberzug b) kann ausserdem zur Verbesserung des Geschmacks sowie zur Erhöhung der Porosität wasserlösliche Zusätze wie Zucker, z.B. Saccharose oder Lactose, oder Aromastoffe enthalten.

Die vorliegende Erfindung betrifft vorzugsweise eine pharmazeutische Zusammensetzung in Form einer Klebetablette, worin a) der hydrophile Tablettenkern den Wirkstoff Baclofen, als quellfähiges Vinylpolymerisat ein Polymeres der Acrylsäure mit einer mittleren Molmasse von ca. $8{,}0 \times 10^5$ bis $1{,}0 \times 10^6$, ein Vinylpyrrolidon-Polymerisat oder ein Vinylpyrrolidon-Vinylacetat-Copolymerisat, als Galactomannan ein kaltwasserlösliches Galactomannan (25° C) mit einer Brookfield-Viskosität grösser als 1'000 [mPa sec] und/oder ein pharmazeutisch annehmbares Wachs enthält und b) der hydrophobe Ueberzug aus Polyäthylenglycol mit filmbildenden Eigenschaften und gegebenenfalls einem Zucker besteht.

Eine weitere bevorzugte Ausführungform betrifft eine pharmazeutische Zusammensetzung in Form einer Klebetablette, worin

a) der hydrophile Tablettenkern den Wirkstoff Baclofen, als quellfähiges Vinylpolymerisat ein Polymeres der Acrylsäure mit einer mittleren Molmasse von ca. $8{,}0 \times 10^5$ bis $1{,}0 \times 10^6$, als Galactomannan ein kaltwasserlösliches Galactomannan (25° C) mit einer Brookfield-Viskosität grösser als 1000 [mPa sec] und ein vollständig oder partiell hydriertes Glycerid, z.B. hydriertes Ricinusöl, und/oder ein pharmazeutisch annehmbares Wachs enthält, und

b) der hydrophobe Ueberzug aus Polyäthylenglycol mit filmbildenden Eigenschaften und gegebenenfalls einem Zucker besteht.

Die vorliegende Erfindung betrifft insbesondere eine pharmazeutische Zusammensetzung in Form einer Klebetablette, worin a) der hydrophile Tablettenkern den Wirkstoff Baclofen, als quellfähiges Vinylpolymerisat ein Polymeres der Acrylsäure mit einer mittleren Molmasse von ca. $8,0 \times 10^5$ bis $1,0 \times 10^6$, als Galactomannan Guar-Gummi und ein vollständig oder hydriertes Glycerid enthält und der hydrophobe Ueberzug b) aus Polyäthylenglycol mit einer mittleren Molmasse grösser als 4'000 und gegebenenfalls Saccharose besteht.

Die pharmazeutische Zusammensetzung der vorliegenden Erfindung eignet sich als Myotonolytikum zur Behandlung von Spastizität bei multipler Sklerose, ferner Muskelspasmen bei Rückenmarkserkrankungen infektiöser oder degenerativer Genese, z.B. bei spastischer Spinalparalyse, amyotropher Lateralsklerose, oder Syringomyelie. Zur Anwendung gelangen die Klebetabletten mit der für Baclofen festgelegten Dosis, z.B. 5, 10 oder 25 mg. Bevorzugt werden 1-2 Tabletten täglich mit 25 mg Wirkstoff verabreicht. Die therapeutische Verwendung der pharmazeutischen Zusammensetzung als Myotonolytikum ist ebenfalls Gegenstand der vorliegenden Erfindung.

Die erfindungsgemässe pharmazeutische Zusammensetzung wird analog bekannten Tablettier- und Beschichtungsverfahren hergestellt. Dieses Verfahren ist dadurch gekennzeichnet, dass man durch Verpressen eines Granulats enthaltend die Bestandteile des Kerns a) oder durch Direktverpressen der Bestandteile, gegebenenfalls unter Zusatz von Gleitmitteln, den hydrophilen Tablettenkern a) herstellt und gegebenenfalls diesen mit Ausnahme der für die Abgabe des Wirkstoffs vorgesehenen freigelassenen Fläche mit dem hydrophoben Ueberzug b) versieht.

Die Herstellung erfolgt vorzugsweise durch Verpressen eines Granulats, welches man z.B. durch Sieben und gegebenenfalls Zerkleinern des Wirkstoffs und gegebenenfalls der Hilfsstoffe, Direktverpressen (Kompatieren) oder Feuchtgranulieren mit einem Lösungsmittel wie Aethanol oder Wasser, Entfernen des Lösungsmittels bzw. Trocknen, gegebenenfalls unter Zusatz von Schmier-oder Gleitmitteln wie Magnesiumstearat, Zerkleinern und nochmaliges Sieben erhält.

Das Verpressen des Granulats zu Tablettenkernen kann in üblichen Tablettiermaschinen erfolgen, z.B. EKO-Korsch-Exzenter-Tablettiermaschinen, Verpressdruck ca. 10 kN. Das Ueberziehen kann durch Lackieren mit einer wässrig-äthanolischen Lösung erfolgen, welche z.B. Polyäthylenglycol und Saccharose gelöst bzw. dispergiert enthält. Vorzugsweise bleibt eine Seite frei von Lacküberzug.

Die pharmazeutische Zusammensetzung kann unterschiedlich geformt und z.B. rund, oval, oblong, zylindrisch u.a. sein und verschiedene Grössen in Abhängigkeit von der Wirkstoffmenge haben. Sie kann ausserdem transparent, farblos oder gefärbt und gegebenenfalls beschriftet sein, um diesem Produkt ein individuelles Aussehen bzw. sofortige Erkennbarkeit zu verleihen. Die Verwendung von Farbstoffen kann sowohl der Hebung des Aussehens als auch der Kennzeichnung des Präparates dienen. Geeignete für die Verwendung in der Pharmazie zugelassene Farbstoffe sind z.B. Carotinoide, Eisenoxide oder Chlorophylle.

| Beispiel 1 | |
|---|---|
| Baclofen (LIORESAL) | 25,00 mg |
| MEYPROGAT 150 | 42,16 mg |
| CARBOPOL 934 P | 22,39 mg |
| Magnesiumstearat | 0,45 mg |
| Wasser (demin.) | 15 ml |

Man siebt das Baclofen, welches bis zu einer durchschnittlichen Korngrosse von 0,5 mm gemahlen ist, MEYPROGAT und CARBOPOL durch ein 0,8 mm Rundsieb und mischt die Bestandteile zehn Minuten lang in einem TURBULA Planetenmischer. Zu dem Gemisch fügt man etwas Wasser hinzu, drückt das angefeuchtete Gemisch durch ein 2,0 mm Rundsieb und trocknet dieses eine Stunde lang bei 40° im Vakuum. Das Granulat mit einer Restfeuchtigkeit von 6,31 % wird durch Drücken durch ein 0,8 mm Rundsieb zerkleinert und nach Zusatz von Magnesiumstearat abschliessend durch das 0,8 mm Rundsieb gedrückt. Man mischt das erhaltene Granulat noch einmal 3 Minuten lang im Planetenmischer und tablettiert dieses mit einer EKO Korsch Exzenter-Tablettiermaschine, Verpressdruck; ca. 10 kN, Stempel: ca. 7,00 mm Durchmesser. Die Tablettenkerne werden auf einer Seite durch Lackieren mit einer Lackmischung bestehend aus 44 g Polyäthylenglycol 8'000, 29 g Saccharose, 52 g demin. Wasser und 22 g Aethanol überzogen.

| Beispiel 2 | |
|---|---|
| Phase I | mg pro Kern |
| Baclofen<br>Meyprogat® 150<br>Cutina® HR (hydriertes Ricinusöl)<br>Carbopol® 934 P | 25,00<br>30,10<br>12,99<br>21,64 |
| Phase II: | |
| Aerosil® 200 | 0,27<br>90,00 |

Die Komponenten der Phase 1 werden durch 0,8 mm Sieb geschlagen und 10 min im Turbula-Mischer gemischt. Die Mischung wird über einen Kompaktor bei 50 kg/cm² Druck kompaktiert; es entstehen weiche, 0,7 mm dicke Plättchen. Die Plättchen werden über einem 0,8 mm Sieb abgemahlen. Es wird zur inneren Phase I über ein 0,8 mm Sieb die äussere Phase II zugegeben und 5 min im Turbula-Mischer gemischt.

Das Granulat wird mit einer Einstempeltablettiermaschine, bei einer Presskraft von 16 kN und unter Verwendung eines Stempels von 7 mm Durchmesser verpresst.

Die hergestellten Klebetabletten haben gute Klebeigenschaften auf dem Gaumen und sind in ca 12-15 h in vivo löslich.


**Ansprüche**

1. Pharmazeutische Zusammensetzung für die Applikation von Baclofen auf den Schleimhäuten der Mundhöhle in Form einer Klebetablette gekennzeichnet durch:

   a) einen hydrophilen Tablettenkern, welcher mit seiner Oberfläche an der Applikationsfläche der Mundschleimhaut haftet und den Wirkstoff Baclofen, oder ein pharmazeutisch verwendbares Salz davon und als Hilfsstoffe ein quellfähiges Vinylpolymerisat, ein Galactomannan und/oder ein pharmazeutisch verwendbares Wachs und/oder ein gegebenenfalls vollständig oder partiell hydriertes Glycerid und gegebenenfalls weitere pharmazeutisch verwendbare Hilfsstoffe enthält, und gegebenenfalls

   b) einen hydrophoben Ueberzug, welcher den Tablettenkern a) mit Ausnahme der zur Abgabe von Baclofen vorgesehenen Fläche umhüllt.

2. Pharmazeutische Zusammensetzung gemäss Anspruch 1, dadurch gekennzeichnet, dass der hydrophile Tablettenkern a) als quellfähiges Vinylpolymerisat ein Acrylsäurepolymerisat mit einer mittleren Molmasse von ca. $8,0 \times 10^5$ bis $1,0 \times 10^6$, ein Vinylpyrrolidon-Polymerisat oder ein Vinylpyrrolidon-Vinylacetat-Copolymerisat enthält.

3. Pharmazeutische Zusammensetzung gemäss Anspruch 2, dadurch gekennzeichnet, dass der hydrophile Tablettenkern a) als quellfähiges Vinylpolymerisat ein Acrylsäurepolymerisat mit einer mittleren Molmasse von ca. $8,0 \times 10^5$ bis $1,0 \times 10^6$ oder Polyvinylpyrrolidon mit einer mittleren Molmasse von ca. 20'000 bis 700'000 enthält.

4. Pharmazeutische Zusammensetzung gemäss Anspruch 1, dadurch gekennzeichnet, dass der hydrophile Tablettenkern a) als Galactomannan ein kaltwasserlösliches Galactomannan (25° C) mit einer Brookfield-Viskosität grösser als 1'000 [mPa sec] (demin. wässrige Lösung bei 25° C, Hydratationszeit 1-24 Stunden, 20 UpM) enthält.

5. Pharmazeutische Zusammensetzung gemäss Anspruch 4, dadurch gekennzeichnet, dass der hydrophile Tablettenkern a) als kaltwasserlösliches Galactomannan Guar-Gummi enthält.

6. Pharmazeutische Zusammensetzung gemäss Anspruch 1, dadurch gekennzeichnet, dass der hydrophile Tablettenkern a) als partiell oder vollständig hydriertes Glycerid hydriertes Ricinusöl enthält.

7. Pharmazeutische Zusammensetzung gemäss Anspruch 1, dadurch gekennzeichnet, dass der hydrophobe Ueberzug aus Polyäthylenglycol mit filmbildenden Eigenschaften und gegebenenfalls einem Zucker besteht.

8. Verfahren zur Herstellung einer pharmazeutischen Zusammensetzung gemäss Anspruch 1, dadurch gekennzeichnet, dass man durch Verpressen eines Granulats enthaltend die Bestandteile des Kerns a) oder durch Direktverpressen dieser Bestandteile, gegebenenfalls unter Zusatz von Gleitmitteln, den hydrophilen

EP 0 376 891 A1

Tablettenkern a) herstellt und gegebenenfalls diesen mit Ausnahme der für die Abgabe des Wirkstoffs vorgesehenen Fläche mit dem hydrophoben Ueberzug b) versieht.

9. Verwendung des Wirkstoffs Baclofen, eines quellfähigen Vinylpolymerisats, eines Galactomannans sowie der im hydrophoben Ueberzug b) enthaltenen Komponenten zur Herstellung einer pharmazeutischen Zusammensetzung gemäss Anspruch 1.

10. Pharmazeutische Zusammensetzung gemäss Anspruch 1, zur Anwendung bei der Behandlung des menschlichen oder tierischen Körpers.

Patentansprüche für folgende Vertragsstaaten: ES,GR

1. Verfahren zur Herstellung einer pharmazeutischen Zusammensetzung für die Applikation von Baclofen auf den Schleimhäuten der Mundhöhle in Form einer Klebetablette gekennzeichnet durch:

a) einen hydrophilen Tablettenkern, welcher mit seiner Oberfläche an der Applikationsfläche der Mundschleimhaut haftet und den Wirkstoff Baclofen, oder ein pharmazeutisch verwendbares Salz davon und als Hilfsstoffe ein quellfähiges Vinylpolymerisat, ein Galactomannan und/oder ein pharmazeutisch verwendbares Wachs und/oder ein gegebenenfalls vollständig oder partiell hydriertes Glycerid und gegebenenfalls weitere pharmazeutisch verwendbare Hilfsstoffe enthält, und gegebenenfalls

b) einen hydrophoben Ueberzug, welcher den Tablettenkern a) mit Ausnahme der zur Abgabe von Baclofen vorgesehenen Fläche umhüllt, dadurch gekennzeichnet, dass man durch Verpressen eines Granulats enthaltend die Bestandteile des Kerns a) oder durch Direktverpressen dieser Bestandteile, gegebenenfalls unter Zusatz von Gleitmitteln, den hydrophilen Tablettenkern a) herstellt und gegebenenfalls diesen mit Ausnahme der für die Abgabe des Wirkstoffs vorgesehenen Fläche mit dem hydrophoben Ueberzug b) versieht.

2. Verfahren zur Herstellung einer pharmazeutischen Zusammensetzung gemäss Anspruch 1, dadurch gekennzeichnet, dass der hydrophile Tablettenkern a) als quellfähiges Vinylpolymerisat ein Acrylsäurepolymerisat mit einer mittleren Molmasse von ca. $8,0 \times 10^5$ bis $1,0 \times 10^6$, ein Vinylpyrrolidon-Polymerisat oder ein Vinylpyrrolidon-Vinylacetat-Copolymerisat enthält.

3. Verfahren zur Herstellung einer pharmazeutischen Zusammensetzung gemäss Anspruch 2, dadurch gekennzeichnet, dass der hydrophile Tablettenkern a) als quellfähiges Vinylpolymerisat ein Acrylsäurepolymerisat mit einer mittleren Molmasse von ca. $8,0 \times 10^5$ bis $1,0 \times 10^6$ oder Polyvinylpyrrolidon mit einer mittleren Molmasse von ca. 20'000 bis 700'000 enthält.

4. Verfahren zur Herstellung einer pharmazeutischen Zusammensetzung gemäss Anspruch 1, dadurch gekennzeichnet, dass der hydrophile Tablettenkern a) als Galactomannan ein kaltwasserlösliches Galactomannan (25° C) mit einer Brookfield-Viskosität grösser als 1'000 [mPa sec] (demin. wässrige Lösung bei 25° C, Hydratationszeit 1-24 Stunden, 20 UpM) enthält.

5. Verfahren zur Herstellung einer pharmazeutischen Zusammensetzung gemäss Anspruch 4, dadurch gekennzeichnet, dass der hydrophile Tablettenkern a) als kaltwasserlösliches Galactomannan Guar-Gummi enthält.

6. Verfahren zur Herstellung einer pharmazeutischen Zusammensetzung gemäss Anspruch 1, dadurch gekennzeichnet, dass der hydrophile Tablettenkern a) als partiell oder vollständig hydriertes Glycerid hydriertes Ricinusöl enthält.

7. Verfahren zur Herstellung einer pharmazeutischen Zusammensetzung gemäss Anspruch 1, dadurch gekennzeichnet, dass der hydrophobe Ueberzug aus Polyäthylenglycol mit filmbildenden Eigenschaften und gegebenenfalls einem Zucker besteht.

8

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| Y | EP-A-0 107 941 (TAKEDA CHEM. IND.)<br>* Seite 1, Zeilen 1,2; Seite 2, Zeilen 21-28; Seite 3, Zeile 21; Seite 9, Zeile 14 - Seite 13, Zeile 15; Patentansprüche 1,5,9,10 *<br>--- | 1-4,8-10 | A 61 K 9/20<br>A 61 K 9/32 |
| Y | CHEMICAL ABSTRACTS, Band 98, Nr. 26, Juni 1983, Seite 382, Zusammenfassung Nr. 221818y, Columbus, Ohio, US; & JP-A-58 43 915 (TEIJIN LTD) 14-03-1983<br>* Zusammenfassung *<br>--- | 1-4,8-10 | |
| Y | EP-A-0 245 952 (RECKITT AND COLMAN PRODUCTS LTD)<br>* Das ganze Dokument *<br>--- | 1-4,8-10 | |
| A | EP-A-0 014 514 (ORION-YHTYMA OY)<br>* Seiten 10-14, Beispiele *<br>--- | 6 | |
| A | FR-A-1 288 725 (ABBOTT LAB.)<br>* Insgesamt *<br>--- | 7 | RECHERCHIERTE SACHGEBIETE (Int. Cl.5) |
| A | EP-A-0 020 777 (TEIJIN LTD)<br>--- | | A 61 K |
| A | FR-A-2 393 577 (ERASMUS UNIVERSITEIT ROTTERDAM)<br>* Seite 2, Zeilen 3-9,31-36 *<br>----- | 1 | |

Der vorliegende Recherchenbericht wurde für alle Patentansprüche erstellt

| Recherchenort | Abschlußdatum der Recherche | Prüfer |
|---|---|---|
| DEN HAAG | 04-04-1990 | BENZ K.F. |